# EUROPÄISCHE PATENTSCHRIFT

(11) **EP 0 557 288 B1**
(45) Veröffentlichungstag und Bekanntmachung des Hinweises auf die Patenterteilung: **13.12.1995**
(21) Anmeldenummer: 91915499.7
(22) Anmeldetag: 02.09.1991
(51) Int. Cl.: G01N 33/543, G01N 30/00

(54) **EINWEGREAKTIONSGEFÄSS FÜR DIE FESTPHASENIMMUNANALYTIK UND VERFAHREN ZUR MESSUNG VON ÜBER IMMUNREAKTIONEN BESTIMMMBARE KOMPONENTEN**
DISPOSABLE REACTOR VESSEL FOR SOLID-PHASE IMMUNE ASSAYS AND PROCESS FOR MEASURING COMPONENTS DETECTABLE BY IMMUNE REACTIONS
REACTEUR JETABLE POUR ANALYSES IMMUNOLOGIQUES EN PHASE SOLIDE ET PROCEDE DE DOSAGE DE COMPOSANTS DETECTABLES PAR REACTION IMMUNITAIRE

(30) Priorität: 17.09.1990 DE 4029460; 09.08.1991 DE 4126436
(43) Veröffentlichungstag der Anmeldung: 01.09.1993
(73) Patentinhaber: ABION Beteiligungs- und Verwaltungsgesellschaft mbH, 52428 Jülich (DE)
(72) Erfinder: ERHARDT, Ursula, D-8174 Benediktbeuern (DE)
(86) Internationale Anmeldenummer: DE9100701
(87) Internationale Veröffentlichungsnummer: WO9205442

(56) Entgegenhaltungen:
- WO-A-86/03589
- DE-A- 3 214 287
- DE-A- 3 733 098
- FR-A- 2 247 728
- FR-A- 2 387 452
- US-A- 4 892 710
- CHEMICAL ABSTRACTS, vol. 110, no. 23, 05 Juni 1989, Columbus, OH (US); H.J. KIM et al., no. 208819#

## Beschreibung

Die vorliegende Erfindung betrifft ein Einwegreaktionsgefäß für die Festphasenimmunanalytik sowie Verfahren, bei denen das Einwegreaktionsgefäß verwendet wird.

Affinitätschromatographie ist eine Technik, die bei der präparativen Reinigung von Biomolekülen breite Anwendung findet. Dabei wird eine spezifische Wechselwirkung des zu bestimmenden Moleküls mit einem dazu komplementären Bindungspartner ausgenutzt. Zur praktischen Durchführung solcher Methoden wird in der Regel eine Probe, die das zu reinigende Biomolekül enthält, auf eine Chromatographiesäule gegeben, die auf einem festen Trägermaterial einen der zueinander komplementären Bindungspartner gebunden enthält. Als Paare komplementärer Bindungspartner sind beispielsweise Enzyme und deren Substrate, Antikorper und Hapten bzw.Antigen, sowie zueinander komplementäre DNA- oder RNA-Einzelstränge zu nennen.

Bei der Immunaffinitätschromatographie wird die Wechselwirkung zwischen einem Antigen bzw. Hapten und dem dazugehörigen Antikörper zur Bestimmung ausgenutzt.

Bei Immuno-Assays unter Verwendung der Affinitätschromatographie wurden bisher im wesentlichen wiederverwendbare Säulen, die mit dem Antikörper oder Antigen (im folgengen als Liganden bezeichnet), gebunden an einen festen Träger, gepackt sind, verwendet. Um die zügige Durchführung des Chromatographievorgangs zu gewährleisten, wird die zu analysierende Probe unter hohem Druck durch die Chromatographiesäule gepreßt. Die Anwendung der Hochdruckflüssigkeitschromatographie (HPLC) im Rahmen von Immuno-Assays wird beispielsweise beschrieben von de Elvis, W.U. und Wilson, G.S. in "Analytical Chemistry 1985," Vol. 57, Seiten 2754-2756, wie auch von Sportsman, J.R. et al in "Analytical Chemistry, 1983", Vol. 55, 771-775. Der Einsatz der HPLC zur Durchführung von Immuno-Assays hat jedoch mehrere Nachteile. So macht diese Technik einerseits den Einsatz aufwendiger Apparaturen notwendig, und andererseits werden an die zur Durchführung der Analyse benötigten Chromatographiesäulen hohe technische Anforderungen gestellt. Die Säulen müssen sowohl den bei der HPLC auftretenden hohen Drücken standhalten, wie auch wiederverwendbar sein, um ein rationelles Arbeiten bei dieser Technik zu ermöglichen. Die Wiederverwendung der Säule erfordert jedoch zusätzliche Maßnahmen, bevor eine erneute Probe auf derselben Säule analysiert werden kann. Wie in der DE-OS 37 11 894 offenbart, werden wiederverwendbare Säulen vor jeder Analyse regeneriert, um den Einfluß mitgeschleppter und nicht ausreichend eluierter Substanzen aus dem vorhergehenden Versuch auszuschalten.

Aus der DE-OS 24 48 411 ist ein Reaktionsgefäß für die Festphasenimmunanalytik bekannt. Das für mehrfache Verwendung ausgelegte Gefäß macht jedoch vor einer quantitativen Bestimmung eine Eichung mit Standards der zu bestimmenden Komponente erforderlich. Weiterhin sind bei dem in dieser Schrift offenbarten Reaktionsgefäß Lange Inkubationszeiten notwendig, empfohlenerweise 6 Stunden bis 2 Tage, um eine ausreichende Bindung der zu bestimmenden Komponente an die auf den Träger aufgebrachte immunologische Reaktivkomponente sicherzustellen.

Die Eindungsreaktion wird jedoch immer vor Erreichen des Bindungsgleichgewichts, das 1 - 2 Tage erfordert, abgebrochen, um ein rationelles Arbeiten zu gewährleisten. Auch dieses frühzeitige Abbrechen der Bindungsreaktion hat zur Folge, daß zur Eichung eine Vergleichsanalytik mit Standardlösungen durchgeführt werden muß.

Aufgabe der vorliegenden Erfindung ist es, ein Einwegreaktionsgefäß für die Festphasenimmunanalytik zur Verfügung zu stellen, das die rasche Durchführung eines Immuno-Assays ermöglicht, das einfach gehandhabt werden kann, und das zum sofortigen Gebrauch, ohne vorherige Kalibrier- oder Regeneriermaßnahmen, geeignet ist.

Diese Aufgabe wird erfindungsgemäß gelöst durch ein oben und unten offenes Einwegreaktionsgefäß für die Festphasenimmunanalytik, mit zwei darin befindlichen, für Flüssigkeiten durchlässigen, porösen Trenneinrichtungen, zwischen denen sich mindestens eine auf ein Trägermaterial aufgebrachte immunologische Reaktivkomponente befindet, wobei das Trägerbettvolumen bis zu ungefähr 600 »l beträgt und wobei die Durchlässigkeit der oberen und/oder unteren Trenneinrichtung in Kombination mit dem Trägerbett die Durchflußgeschwindigkeit der Flüssigkeiten bestimmt.

Eine weitere Aufgabe besteht darin, rasch durchführbare Verfahren zur Bestimmung von über eine Immunreaktion bestimmbaren Komponenten zur Verfügung zu stellen.

Diese Aufgabe wird gelöst durch ein Verfahren, bei dem auf das erfindungsgemäße Einwegreaktionsgefäß eine zu analysierende Probe gegeben wird und die zu bestimmende Komponente in der Probe von der komplementären immunologischen Reaktivkomponente in dem Einwegreaktionsgefäß zurückgehalten wird, wobei sich ein Bindungsgleichgewicht einstellt. Die zu bestimmende Komponente wird dann
a) in an sich bekannter Weise eluiert und bestimmt, oder
b) im Einwegreaktionsgefäß in an sich bekannter Weise über Verstärkerreaktionen mit üblichen immunologischen Markern markiert und dann bestimmt oder
c) über eine zu der zu bestimmenden Komponente konkurrierende markierte Verbindung bestimmt.

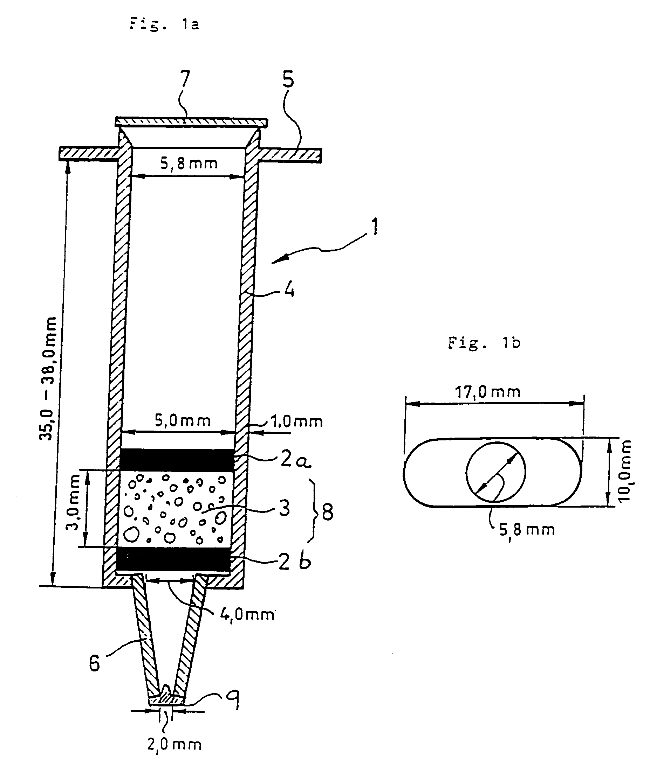

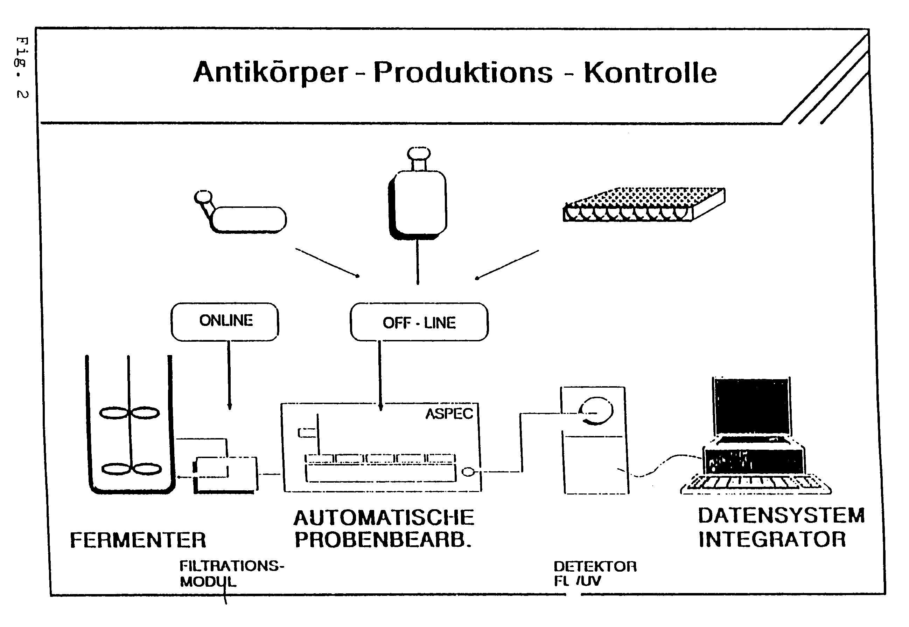

Die Erfindung wird nachfolgend mit Bezug auf die Fig. 1 und 2 näher erläutert, in denen zeigen:
- Fig.1a: einen Längsschnitt durch ein erfindungsgemäßes Einwegreaktionsgefäß 1 mit zwei darin enthaltenen Trenneinrichtungen 2a und 2b, zwischen denen sich das Trägermaterial mit der immunologischen Reaktivkomponente 3 befindet,
- Fig.1b: eine Draufsicht auf das Einwegreaktionsgefäß 1 und
- Fig. 2: eine schematische Darstellung eines On-line-Systems zur Antikörper-Produktionskontrolle.

Die in den Figuren 1a und 1b wiedergegebenen Abmessungen sind lediglich beispielhaft.

Das erfindungsgemäße Einwegreaktionsgefäß 1 sowie das Verfahren, bei dem das Einwegreaktionsgefäß 1 verwendet wird, bringen im Vergleich zu bekannten Reaktionsgefäßen bzw. Verfahren erhebliche Vorteile mit sich.

Das erfindungsgemäße Einwegreaktionsgefäß 1 erlaubt eine rasche Durchführung der quantitativen Bestimmung von Komponenten, die über Immunreaktionen bestimmbar sind. Die Hanchabung des Reaktionsgefäßes 1 ist denkbar einfach und erfordert nicht den Einsatz aufwendiger Apparaturen wie z.B. von HPLC-Apparaturen. Weiterhin werden ausgezeichnete Ergebnisse mit dem genannten Einwegreaktionsgefäß 1 erhalten bei einem äußerst geringen Verbrauch an Trägermaterial 3 mit der darauf aufgebrachten immunologischen Reaktivkomponente (im folgenden als Trägerbett bezeichnet). Ein Einwegreaktionsgefäß 1 mit einem Trägerbettvolumen 3, das ist das sich zwischen der oberen und unteren Trenneinrichtung 2a und 2b befindliche Volumen, von vorzugsweise 50 »l wird in dem erfindungsgemäßen Verfahren bevorzugt verwendet. Das geringe Trägerbettvolumen ermöglicht die Verwendung geringer Mengen an Elutionsflüssigkeit, was wiederum zur Folge hat, daß der Verdünnungseffekt durch die Elution gering bleibt, und somit die Nachweisgrenze im Vergleich zu anderen Verfahren, bei denen größere Elutionsvolumina notwendig sind, nicht verschlechtert wird.

Weiterhin ermöglicht das erfindungsgemäße Reaktionsgefäß 1 die quantitative Bestimmung im Bindungsgleichgewicht (Endpunktbestimmung) zwischen zu bestimmender Komponente und der immunologischen Reaktivkomponente, wobei sich das Bindungsgleichgewicht bei Verwendung des erfindungsgemäßen Einwegreaktionsgefäßes 1 bereits innerhalb einer Inkubationszeit von einer Minute einstellt. Das rasche Einstellen des Bindungsgleichgewichts wird durch die obere und/oder untere Trenneinrichtung 2a bzw. 2b, das geringe Trägerbettvolumen 8 sowie die hohe Beladung des Trägermaterials mit der immunologischen Reaktivkomponente ermöglicht. Die obere und/oder untere Trenneinrichtung sorgt in Verbindung mit dem gewählten Trägerbettvolumen für einen kontrollierten Durchfluß, und die hohe Beladungsdichte des Trägermaterials mit der Reaktivkomponente hat zur Folge, daß die zu bestimmende Komponente innerhalb von ca. 1 min nahezu quantitativ an die Reaktivkomponente bindet, bevor die durchfließende Flüssigkeit aus dem Trägerbettvolumen austritt. Dadurch erübrigt sich eine Vergleichsanalytik mit Standardlösungen.

Eine schnelle Analyse von Proben wird weiterhin deshalb ermöglicht, weil das Einwegreaktionsgefäß 1 den sofortigen Gebrauch erlaubt, da das darin enthaltene Trägermaterial 3 mit der immunologischen Reaktivkomponente standardisierbar und konfektionierbar ist und das Einwegreaktionsgefäß 1 vor der Verwendung nicht geeicht werden muß.

Da das Einwegreaktionsgefäß 1 die rasche Analyse von Proben erlaubt, ohne den Einsatz der HPLC, muß das Trägermaterial 3 nicht druckstabil sein. Als Trägermaterial 3 kommen alle herkömmlichen, für die Affinitätschromatographie verwendbaren Materialien in Betracht. Bevorzugte Trägermaterialien sind polymere Zucker, Kunststoffe oder Silikate, besonders bevorzugt ist Agarose.

Die auf das Trägermaterial 3 aufgebrachte immunologische Reaktivkomponente kann sowohl kovalent wie auch adsorptiv an das Trägermaterial 3 gebunden sein. Die immunologische Reaktivkomponente kann ausgewählt werden aus der Gruppe umfassend Haptene, Antigene und Antikörper. Als Antikörper können sowohl polyklonale wie auch monoklonale Antikörper verwendet werden.

Bei einer bevorzugten Ausführungsform des Einwegreaktionsgefäßes 1 befindet sich das Trägermaterial 3 mit der immunologischen Reaktivkomponente zwischen zwei Trenneinreichungen, wobei die obere und/oder untere Trenneinrichtung 2a bzw. 2b eine poröse Fritte ist. Die Porosität der oberen und/oder unteren Fritte 2a bzw. 2b beträgt ungefähr 0,05 »m bis ungefähr 100 »m, vorzugsweise ungefähr 0,2 »m bis ungefähr 100 »m, besonders bevorzugt ist eine Porosität von 2 »m. Die Materialien für die Fritte 2a bzw. 2b sind ausgewählt aus der Gruppe umfassend Kunststoffe, Metalle und Glas, bevorzugt ist Kunststoff, besonders bevorzugt sind Polyethylen und Teflon. Bei einer weiteren bevorzugten Ausführungsform sind die obere und untere Trenneinrichtung 2a und 2b zwei gleiche poröse Fritten.

Bei einer weiteren bevorzugten Ausführungsform beträgt die Schichtdicke der oberen und/oder unteren Fritte ungefähr 0,05 mm bis ungefähr 2 mm, bevorzugt ungefähr 0,2 bis ungefähr 2 mm, ganz besonders bevorzugt ist eine Schichtdicke von ungefähr 1 mm.

Da das Einwegreaktionsgefäß 1 in dem erfindungsgemäßen Verfahren nicht hohen Drücken ausgesetzt werden muß, braucht das Gefäßmaterial 4 nicht druckstabil zu sein. Es kommen somit außer den druckstabilen Materialien, wie Metalle, andere nicht druckstabile Materialien, wie Glas, Naturstoffe und Kunststoff, für die Herstellung des Einwegreaktionsgefäßes 1 in Betracht. Bevorzugte Kunststoffe sind Polyethylen, Polypropylen und/oder Polystyrol und bevorzugte Metalle sind poröses Aluminium und Edelstahl.

Zweckmäßigerweise ist das Einwegreaktionsgefäß 1 an einem Ende mit einem Wulst 5 versehen, der das Einpassen des Reaktionsgefäßes in Halte- und Transportvorrichtungen zur automatischen Weiterbewegung ermöglicht. Dies ist erforderlich für den Einsatz in automatischen Probenbearbeitungsvorrichtungen. Weiterhin ist es zweckmäßig, daß das Einwegreaktionsgefäß 1 an dem einen Wulst 5 tragenden gegenüberliegenden Ende einen Anschluß mit einem kleineren Durchmesser als dem Gefäßdurchmesser besitzt, vorzugsweise einen Anschluß, der eine Steckverbindung mit einem weiteren Einwegreaktionsgefäß ermöglicht. Dieser erleichtert u.a. das In-Reihe-Schalten mehrerer Einwegreaktionsgefäße 1, wobei der Auslauf 6 des ersten Gefäßes mit dem Einlauf des zweiten Gefäßes durch einfaches Zusammenstecken der beiden Gefäße verbunden werden in Art einer Male-female-Steckverbindung.

Weiterhin ist das Einwegreaktionsgefäß 1 durch Abdeckungen 7 und 9 abschließbar.

Das erfindungsgemäße Verfahren unter Verwendung des obigen Einwegreaktionsgefäßes 1 zeichnet sich u.a. dadurch aus, daß die Durchführung im Vergleich zu bisherigen Verfahren stark vereinfacht ist und es eine rasche quantitative und qualitative Bestimmung erlaubt. Dieses wird zum Teil deshalb ermöglicht, weil die Bestimmung der zu bestimmenden Komponente keine vorherige Kalibrierung oder Regenerierung des in dem Einwegreaktionsgefäß 1 verwendeten Trägermaterials erfordert und keine Vergleichsreihen mit Standardlösungen durchgeführt werden müssen. Die einfache Handhabung ergibt sich aus der Möglichkeit, Proben und weitere Lösungen mit üblichen Laborhilfsmitteln oder einem Pipettierautomaten aufzubringen.

Neben manuellen Verfahren werden auch automatisierte Verfahren durch den Einsatz des erfindungsgemäßen Reaktionsgefäßes 1 verbessert. Bei solchen Verfahren erfolgt das Aufbringen der Probe und die weiteren Verfahrensschritte durch eine automatische Probenbearbeitungsvorrichtung. Eine solche Probenbearbeitungsvorrichtung findet beispielsweise bei der Produktionskontrolle von in einem Fermenter hergestellten Antikörpern durch ein Online-System zur Prozeßkontrolle statt. Bei einem solchen Verfahren erfolgt die automatische Probenaufbringung in zeitlichen Abständen von etwa 6 bis 8 Stunden, und die in dem Fermentationsmedium enthaltene Antikörperkonzentration kann so kontinuierlich über einen längeren Zeitraum mit Hilfe des Einwegreaktionsgefäßes 1 rasch und einfach bestimmt werden. Eine schematische Darstellung eines Systems zur Antikörperproduktionskontrolle, bei dem das erfindungsgemäße Einwegreaktionsgefäß 1 eingesetzt werden kann, ist in Fig. 2 gezeigt. Dabei befindet sich das Einwegreaktionsgefäß 1 in der automatischen Probenbearbeitungsvorrichtung.

Das erfindungsgemäße Verfahren kann rasch durchgeführt werden, ohne daß das Einwegreaktionsgefäß 1 nach dem Aufbringen der Probe hohen Drücken ausgesetzt werden muß. Jedoch können zur schnelleren Durchführung des Verfahrens leichte Sog- oder Druckkräfte, wie sie z.B. durch Zentrifugation oder Pumpvorrichtungen zu erzeugen sind, angewendet werden.

Falls erforderlich, können bei den erfindungsgemäßen Verfahren auch mehrere Einwegreaktionsgefäße 1 hintereinander oder parallel zueinander geschaltet werden. Dies erlaubt z.B. die gleichzeitige Bestimmung mehrerer Parameter in einer einzigen Probe bzw. die gleichzeitige Analyse mehrerer Proben.

Das Hintereinanderschalten mehrerer Einwegreaktionsgefäße ist z.B. bei der Durchführung von Allergietests besonders vorteilhaft. Dabei wird auf verschiedene Säulen jeweils ein unterschiedliches Antigen (Allergen) aufgebracht, die Säulen werden dann in Reihe geschaltet, und die Probe, enthaltend den die allergische Reaktion vermittelnden Antikörper der Klasse IgE wird auf das erste Reaktionsgefäß aufgetragen. Die Probe durchläuft sukzessiv die verschiedenen, mit unterschiedlichen Allergenen bestückten Einwegreaktionsgefäße 1. In dem Reaktionsgefäß, in dem das die Allergie hervorrufende Antigen vorhanden ist, wird das in der Probe enthaltene IgE gebunden und kann dort z. B. über Fluoreszensdetektion nachgewiesen werden. Somit kann eine einzige Probe gleichzeitig mit einer Vielzahl verschiedener, potentieller Allergene ausgetestet werden.

Die folgenden Beispiele erläutern die Erfindung.

### Beispiel 1

Manuelle Durchführung des erfindungsgemäßen Verfahrens

Ein Einwegreaktionsgefäß 1 aus Polypropylen, wie in Fig. 1 gezeigt, enthielt die folgenden Komponenten:

Zwischen zwei Fritten 2a und 2b aus Teflon (Porengröße etwa 2 »m, Schichtdicke etwa 1 mm) befinden sich 100 »l einer Suspension, enthaltend handelsübliche, Cyanbromid aktivierte, quervernetzte Agarose mit kovalent gebundenem polyklonalem Maus-Anti-Human-IgG in 0,5 M NaCl. 100 »l der Suspension ergeben letztendlich ein Trägerbett-Volumen 8 von 50 »l. Die Bindungskapazität pro ml Trägerbett beträgt ca. 700 ug Human-IgG.

Als Equilibrierungspuffer (EP) wurde verwendet: 10 mM Phosphatpuffer pH 7,2, 150 mM NaCl; als Elutionspuffer (EL): 1 M Propionsäure; als Zellkulturmedium (ZM): RPMI-Medium mit 10% fötalem Kälberserum (FKS).

Zunächst wurden 50 »l Probe, enthaltend 0,5 »g bis 50 »g Human-IgG in ZM, auf das Reaktionsgefäß gegeben. Es wurde mit 1 ml EP gewaschen, das Eluat wurde verworfen, und anschließend wurde mit 0,5 ml EL eluiert. Das Eluat wurde in einer Küvette zur Fluoreszenzdetektion aufgefangen und im Detektor gemessen. Zur quantitativen Bestimmung des Gehalts der Probe an zu bestimmendem Human-IgG wurde zuvor eine Eichkurve erstellt, bei der Human-IgG in 1 M Propionsäure direkt im Detektor gemessen wurde. Es wurden IgG-Konzentrationen von 0 »g/ml bis 1000 »g/ml, vorzugsweise von 0 »g/ml bis 100 »g/ml, zur Erstellung der Eichkurve eingesetzt und
a) die Extinktion im UV-Detektor im Bereich von 250 - 300 nm, bevorzugt bei 280 nm, oder
b) die relative Fluoreszensintensität im Fluoreszensdetektor bei einer Anregungswellenlänge von 250 - 290 nm und einer Emissionswellenlänge von > 320 nm ermittelt.

Bei Verwendung von Markermolekülen müssen die Detektionswellenlängen dem Marker entsprechend angepaßt werden. Als Leerwert, der von dem für die Probe ermittelten Meßwert abgezogen wurde, diente der mit dem Zellkulturmedium alleine erzielte Wert.

Zur praktischen Durchführung der Bestimmung kann der Probe- bzw. Eluat-Durchfluß durch das Reaktionsgefäß 1 im einfachsten Fall aufgrund der Schwerkraft erfolgen. Alternativ dazu kommt für die Beschleunigung des Durchflusses in Frage:
a) Druck: Dieser kann im einfachsten Fall unter Verwendung einer Einmalspritze, die über dem Luer-Lock-Anschluß mit dem Reaktionsgefäß 1 verbunden ist, erzeugt werden. Andererseits kann auch mit anderen Hilfsmitteln, wie z.B. einem von Hand bedienbaren Dispensor, der Druck erzeugt werden.
b) Vakuum: Dieses kann durch den Anschluß einer herkömmlichen Saugpumpe am Ausgang des Reaktionsgefäßes erzeugt werden.
c) Zentrifugalkraft: Bei dieser Variante können die Probe-, Wasch- und Elutionsflüssigkeit z.B. in abgeschlossenen Kammersystemen, die über Sollbruchstellen verfügen, eingebracht werden, und durch das Anlegen einer geeigneten Zentrifugalkraft können die Flüssigkeiten bei Erreichen einer gegebenen Zentrifugalkraft durch die Sollbruchstellen auf das Reaktionsgefäß gelangen.

### Beispiel 2

Vollautomatisierte Durchführung des erfindungsgemäßen Verfahrens.

Die zur Durchführung verwendete Apparatur enthielt folgende Einzelkomponenten:
einen HPLC-Probengeber (ABIMED ASPEC System), Einwegreaktionsgefäße 1, einen Fluoreszenz- oder UV-Detektor mit Durchflußzelle und eine Computereinheit zur Datenauswertung.

Die Analyse wurde wie folgt durchgeführt:

Die Probenahme aus der Zellkultur erfolgte über gängige Probenahmesysteme. Nach Abtrennung der Zellen über Filter (im ASPEC-System) und Positionieren des Racks mit den Einwegreaktionsgefäßen 1 über der Abfallposition erfolgte die Aufgabe der Probe, erneutes Waschen mit Equilibrierungspuffer und anschließend Positionieren des Racks mit den Reaktionsgefäßen in der Ablöseposition. Das Ablösen der zu bestimmenden Komponente (Eluieren) erfolgte in einem Schritt. Die zu bestimmende Komponente wurde in der Elutionslösung zum Durchflußdetektor transportiert und gemessen. Die eingesetzten Volumina entsprechen den in Beispiel 1 angegebenen.

## Patentansprüche

1. Oben und unten offenes Einwegreaktionsgefäß (1) für die Festphasenimmunanalytik mit zwei darin befindlichen, für die zu bestimmende Komponente enthaltende Flüssigkeiten durchlässigen, porösen Trenneinrichtungen (2a) und (2b), zwischen denen sich mindestens eine auf ein Trägermaterial (3) aufgebrachte immunologische Reaktivkomponente mit einer derartigen Beladungsdichte befindet, daß die zu bestimmende Komponente quantitativ an die Reaktivkomponente bindet, bevor die durchfließende Flüssigkeit aus dem Trägerbett austritt, wobei das Trägerbettvolumen (8) bis zu 600 »l beträgt, und wobei die Durchlässigkeit der oberen und/oder unteren Trenneinrichtung (2a) bzw. (2b) die Durchflußgeschwindigkeit der Flüssigkeiten bestimmt.

2. Einwegreaktionsgefäß nach Anspruch 1, **dadurch gekennzeichnet**, daß das Trägermaterial (3) mit der immunologischen Reaktivkomponente standardisiert und konfektioniert ist, was den sofortigen Gebrauch des Einwegreaktionsgefäßes (1) erlaubt.

3. Einwegreaktionsgefäß nach einem der Ansprüche 1 oder 2, **dadurch gekennzeichnet**, daß das Trägermaterial (3) ausgewählt wird aus der Gruppe, umfassend polymere Zucker, Kunststoffe oder Silikate.

4. Einwegreaktionsgefäß nach Anspruch 3, **dadurch gekennzeichnet**, daß der polymere Zucker Agarose ist.

5. Einwegreaktionsgefäß nach Anspruch 1, **dadurch gekennzeichnet,** daß die immunologische Reaktivkomponente kovalent oder adsorptiv an das Trägermaterial (3) gebunden ist.

6. Einwegreaktionsgefäß nach Anspruch 5, **dadurch gekennzeichnet,** daß die immunologische Reaktivkomponente ausgewählt wird aus der Gruppe, umfassend Hapten, Antigen und Antikörper.

7. Einwegreaktionsgefäß nach Anspruch 6, **dadurch gekennzeichnet,** daß der Antikörper polyklonal ist.

8. Einwegreaktionsgefäß nach Anspruch 6, **dadurch gekennzeichnet**, daß der Antikörper monoklonal ist.

9. Einwegreaktionsgefäß nach Anspruch 1, **dadurch gekennzeichnet**, daß die obere und/oder untere Trenneinrichtung (2a, 2b) eine poröse Fritte oder eine Membran ist.

10. Einwegreaktionsgefäß nach Anspruch 9, **dadurch gekennzeichnet,** daß die Fritte (2a, 2b) eine Porosität von 0,2 »m bis 100 »m.

11. Einwegreaktionsgefäß nach Anspruch 9, **dadurch gekennzeichnet**, daß die Fritte (2a, 2b) eine Schichtdicke von 0,05 mm bis 2 mm aufweist.

12. Einwegreaktionsgefäß nach Anspruch 11, **dadurch gekennzeichnet,** daß die Schichtdicke 1 mm beträgt.

13. Einwegreaktionsgefäß nach einem der Ansprüche 9 bis 12, **dadurch gekennzeichnet**, daß die Fritte (2a, 2b) aus Kunststoff, Metall oder Glas besteht.

14. Einwegreaktionsgefäß nach Anspruch 13, **dadurch gekennzeichnet**, daß der Kunststoff Polyethylen oder Teflon und das Metall poröses Aluminium oder Edelstahl ist.

15. Einwegreaktionsgefäß nach einem der vorhergehenden Ansprüche 1 bis 14, **dadurch gekennzeichnet**, daß das Gefäßmaterial (4) ausgewählt wird aus der Gruppe, umfassend Kunststoffe, Metalle und Naturstoffe.

16. Einwegreaktionsgefäß nach Anspruch 15, **dadurch gekennzeichnet,** daß der Kunststoff Polyethylen, Polypropylen und/oder Polystyrol ist.

17. Einwegreaktionsgefäß nach einem der vorhergehenden Ansprüche 1 bis 16, **dadurch gekennzeichnet**, daß das Reaktionsgefäß (1) an einem Ende einen Wulst (5) trägt, der das Einpassen des Reaktionsgefäßes in Halte- und Transporteinrichtungen zur automatischen Weiterbewegung ermöglicht.

18. Einwegreaktionsgefäß nach einem der vorhergehenden Ansprüche 1 bis 17, **dadurch gekennzeichnet**, daß das Reaktionsgefäß (1) an dem einen Wulst (5) tragenden gegenüberliegenden Ende einen Anschluß (6) mit einem kleineren Durchmesser als dem Gefäßdurchmesser besitzt.

19. Einwegreaktionsgefäß nach Anspruch 18, **dadurch gekennzeichnet**, daß die gegenüberliegenden Enden des Reaktionsgefäßes ein Zusammenkoppeln in Art einer Male-Female-Steckverbindung ermöglichen zum Hintereinanderschalten mehrerer Einwegreaktionsgefäße.

20. Einwegreaktionsgefäß nach einem der vorhergehenden Ansprüche 1-19, **dadurch gekennzeichnet,** daß das Trägerbettvolumen (8) 50 »l beträgt.

21. Verfahren zur Bestimmung von über eine Immunreaktion bestimmbaren Komponenten, **dadurch gekennzeichnet,** daß auf ein Einwegreaktionsgefäß nach einem der Anspruche 1 bis 20 eine zu analysierende Probe gegeben wird, die zu bestimmende Komponente in der Probe von der komplementären immunologischen Reaktivkomponente in dem Einwegreaktiongsgefäß zurückgehalten wird, und die zu bestimmende Komponente
a) eluiert und bestimmt wird, oder
b) im Einwegreaktionsgefäß über Verstärkerreaktionen mit immunologischen Markern markiert und dann bestimmt wird, oder
c) über eine zu der zu bestimmenden Komponente konkurrierende markierte Verbindung bestimmt wird.

22. Verfahren nach Anspruch 21, **dadurch gekennzeichnet,** daß die Bestimmung ohne vorherige Kalibrierung oder Regenerierung des Trägermaterials und der darauf aufgebrachten Reaktivkomponente durchgeführt wird.

23. Verfahren nach Anspruch 21 oder 22, **dadurch gekennzeichnet**, daß das Aufbringen der Probe und weiterer Lösungen mit üblichen Laborhilfsmitteln oder einem Pipettierautomaten erfolgt.

24. Verfahren nach einem der Ansprüche 21 oder 22, **dadurch gekennzeichnet**, daß das Aufbringen der Probe und die weiteren Verfahrensschritte durch eine automatische Probenbearbeitungsvorrichtung durchgeführt werden.

25. Verfahren nach Anspruch 24, **dadurch gekennzeichnet,** daß die automatische Probenaufbringung in einem On-line-Prozeßkontroll-System erfolgt.

26. Verfahren nach einem der Ansprüche 21 bis 25, **dadurch gekennzeichnet**, daß zur schnelleren Durchführung des Verfahrens das Einwegreaktionsgefäß nach dem Aufbringen der Probe leichten Sog- oder Druckkräften ausgesetzt wird.

27. Verfahren nach einem der Ansprüche 21 bis 26, **dadurch gekennzeichnet**, daß mehrere Einwegreaktionsgefäße hintereinander oder parallel geschaltet werden.

28. Verfahren nach Anspruch 27, **dadurch gekennzeichnet**, daß damit Allergene bestimmt werden.

## Claims

1. A disposable reaction vessel (1), which is open at the top and at the bottom, for solid-phase immune assays having located therein two porous partitioning devices (2a) and (2b) which are permeable for liquids containing components to be analysed and between the said partitioning devices at least one immunological reactive component applied to a carrier material (3), which reactive component is loaded with a density such that the component to be determined quantitatively binds to the reactive component prior to the through-flowing liquid exiting from the carrier bed, wherein the carrier bed volume (8) amounts to up to 600 »l and wherein the permeability of the upper and/or lower partitioning device (2a) und (2b) determines the rate of through flow of the liquids.

2. A disposable reaction vessel according to claim 1, characterised in that the carrier material (3) is standardized and fabricated with the immunological reactive component, which enables the disposable reaction vessel (1) to be put into immediate use.

3. A disposable reaction vessel according to either one of claims 1 or 2, characterised in that the carrier material (3) is selected from the group which comprises polymeric sugar, plastic material and silicate.

4. A disposable reaction vessel according to claim 3, characterised in that the polymeric sugar is agarose.

5. A disposable reaction vessel according to claim 1, characterised in that the immunological reactive component is bound in a covalent or adsorptive manner to the carrier material (3).

6. A disposable reaction vessel according to claim 5, characterised in that the immunological reactive component is selected from the group which comprises hapten, antigen and antibodies.

7. A disposable reaction vessel according to claim 6, characterised in that the antibody is polyclonal.

8. A disposable reaction vessel according to claim 6, characterised in that the antibody is monoclonal.

9. A disposable reaction vessel according to claim 1, characterised in that the upper and/or the lower partitioning device (2a, 2b) is porous frit or a membrane.

10. A disposable reaction vessel according to claim 9, characterised in that the frit (2a, 2b) has a porosity of 0.2 »m to 100 »m.

11. A disposable reaction vessel according to claim 9, characterised in that the frit (2a, 2b) has a thickness of the layer of 0,05 mm to 2 mm.

12. A disposable reaction vessel according to claim 11, characterised in that the thickness of the layer amounts to 1 mm.

13. A disposable reaction vessel according to any one of claims 9 to 12, characterised in that the frit (2a, 2b) is made from plastic material, metal or glass.

14. A disposable reaction vessel according to claim 13, characterised in that the plastic material is polyethylene or Teflon and the metal is a porous aluminium or high-quality steel.

15. A disposable reaction vessel according to any one of the preceding claims 1 to 14, characterised in that the vessel material (4) is selected from the group which comprises plastic materials, metals and natural materials.

16. A disposable reaction vessel according to claim 15, characterised in that the plastic material is polyethylene, polypropylene and/or polystyrene.

17. A disposable reaction vessel according to any one of the preceding claims 1 to 16, characterised in that the reaction vessel (1) carries at one end a projection (5) by means of which the reaction vessel is able to fit in the holding and transportation devices for the purpose of automatic further movement.

18. A disposable reaction vessel according to any one of the preceding claims 1 to 17, characterised in that the reaction vessel (1) comprises at the end opposite to the end bearing a projection (5) a connection (6) which has a smaller diameter than that of the vessel.

19. A disposable reaction vessel according to claim 18, characterised in that the opposite ends of the reaction vessel form a coupling in a type of male-female plug-in connection for the purpose of connecting in series a plurality of disposable reaction vessels.

20. A disposable reaction vessel according to any one of the preceding claims 1 to 19, characterised in that the carrier bed volume (8) amounts to 50 »l.

21. A method for the determination of components which can be determined by way of an immune reaction, characterised in that a sample to be analysed is added to a disposable reaction vessel according to any one of claims 1 to 20, the component to be determined in the sample is retained by the complementary immunological reactive component in the disposable reaction vessel and the component to be determined is:
a) eluted and determined, or
b) in the disposable raction vessel it is marked by way of amplification reactions with immunological markers and then determined, or
c) it is determined by way of a marked compound which competes with the component to be determined.

22. A method according to claim 21, characterised in that the determination is carried out without previously calibrating or regenerating the carrier material and the reactive component applied thereto.

23. A method according to claim 21 or 22, characterised in that the sample and other solutions are applied using conventional laboratory equipment or an automatic pipetting device.

24. A method according to any one of claims 21 or 22, characterised in that the sample is applied and the other method steps are carried out by virtue of an automatic sample processing device.

25. A method according to claim 24, characterised in that the sample is applied automatically in an on-line-process control-system.

26. A method according to any one of claims 21 to 25, characterised in that in order to carry out the method more rapidly the disposable reaction vessel is subjected to light pulling or pushing forces after the sample has been applied.

27. A method according to any one of claims 21 to 26, characterised in that a plurality of disposable reaction vessels are connected in series or parallel to each other.

28. A method according to claim 27, characterised in that in this way allergenes are determined.

## Revendications

1. Réacteur jetable (1) ouvert à ses extrémités supérieure et inférieure pour analyses immunologiques en phase solide contenant deux dispositifs de séparation (2a) et (2b) poreux permettant le passage des liquides contenant les constituants à doser et entre lesquels est disposé un lit de réactif immunologique déposé sur un matériau support (3) présentant une densité de chargement telle qu'il permette au constituant à doser de se lier, quantitativement, au réactif avant que le liquide traversant le volume du lit-support ne s'en échappe, le volume du lit-support (8) pouvant atteindre jusqu'à 600 »l et la perméabilité des dispositifs de séparation supérieur (2a) et/ou inférieur (2b) déterminant la vitesse d'écoulement des liquides.

2. Réacteur jetable selon la revendication 1, caractérisé en ce que le matériau support (3) est normalisé et formé du réactif immunologique, ce qui permet l'utilisation immédiate du réacteur jetable (1).

3. Réacteur jetable selon l'une des revendications 1 ou 2, caractérisé en ce que le matériau support (3) est un matériau choisi dans le groupe comprenant, des matières synthétiques, des silicates ou des sucres polymères.

4. Réacteur jetable selon la revendication 3, caractérisé en ce que le sucre polymère est l'agarose.

5. Réacteur jetable selon la revendication 1, caractérisé en ce que le réactif immunologique est lié au support (3) par covalence ou adsorption.

6. Réacteur jetable selon la revendication 5, caractérisé en ce que le réactif immunologique est choisi dans le groupe comprenant des déterminants antigéniques, des antigènes et des anticorps.

7. Réacteur jetable selon la revendication 6, caractérisé en ce que l'anticorps est polyclonal.

8. Réacteur jetable selon la revendication 6, caractérisé en ce que l'anticorps est monoclonal.

9. Réacteur jetable selon la revendication 1, caractérisé en ce que les dispositifs de séparation (2a, 2b) supérieur et/ou inférieur sont formés d'un élément poreux fritté ou d'une membrane.

10. Réacteur jetable selon la revendication 9, caractérisé en ce que l'élément fritté (2a, 2b) présente une porosité de 0,2 »m à 100 »m.

11. Réacteur jetable selon la revendication 9, caractérisé en ce que l'élément fritté (2a, 2b) présente une épaisseur comprise entre 0,05 mm et 2 mm.

12. Réacteur jetable selon la revendication 11, caractérisé en ce que l'épaisseur est de 1 mm.

13. Réacteur jetable selon l'une des revendications 9 à 12, caractérisé en ce que l'élément fritté (2a, 2b) est composé de matières synthétiques, de métal ou de verre.

14. Réacteur jetable selon la revendication 13, caractérisé en ce que le matériau synthétique est du polyéthylène ou du téflon et en ce que le métal est de l'aluminium poreux ou de l'acier fin.

15. Réacteur jetable selon l'une des revendications qui précèdent 1 à 14, caractérisé en ce que le matériau (4) du réacteur est choisi dans le groupe comprenant des matériaux synthétiques, des métaux et des matériaux naturels.

16. Réacteur jetable selon la revendication 15, caractérisé en ce que le matériau synthétique est le polyéthylène, le polypropylène et/ou le polystyrol.

17. Réacteur jetable selon l'une des revendications 1 à 16, caractérisé en ce que le réacteur porte à une de ses extrémités une collerette (5) qui rend possible l'adaptation du réacteur à des dispositifs d'arrêt et de transport en vue d'un déplacement automatique ultérieur.

18. Réacteur jetable selon la revendication 1 des revendications précédentes 1 à 17, caractérisé en ce que le réacteur (1) présente, à l'extrémité opposée à celle qui comporte une collerette (5), un tube de raccordement (6) dont le diamètre est inférieur à celui du réacteur.

19. Réacteur jetable selon la revendication 18, caractérisé en ce que les extrémités opposées du réacteur comportent des moyens de couplage du type mâle-femelle permettant de connecter en cascade plusieurs réacteurs jetables.

20. Réacteur jetable selon l'une des revendications précédentes 1 à 19, caractérisé en ce que le volume (8) du lit-support est de 50 »l.

21. Procédé de détermination de constituants déterminables par une réaction immunologique, caractérisé en ce que l'échantillon à analyser est introduit dans le réacteur jetable selon l'une des revendications 1 à 20, en ce que le constituant à déterminer dans l'échantillon est retenu dans le réacteur jetable par rapport au constituant réactif complémentaire immunologique et en ce que le constituant à doser,
- a) est élué et dosé, ou
- b) est marqué dans le réacteur jetable par des agents de marquage immunologiques via des réactions d'amplification et est ensuite dosé, ou
- c) est dosé par l'intermédiaire d'un composé marqué antagoniste du composant à doser.

22. Procédé selon la revendication 21, caractérisé en ce que l'on effectue la détermination sans étalonnage préalable ou régénération du matériau support et des constituants réactifs qui y ont été introduits.

23. Procédé selon la revendication 21 ou la revendication 22, caractérisé en ce que l'introduction de l'échantillon et des autres solutions se fait à l'aide des moyens de laboratoires usuels ou d'une pipette automatique.

24. Procédé selon l'une des revendications 21 ou 22, caractérisé en ce que l'introduction de l'échantillon et les étapes ultérieures du procédé sont réalisées à l'aide d'un dispositif automatique de traitement d'échantillons.

25. Procédé selon la revendication 24, caractérisé en ce que l'introduction automatique de l'échantillon se fait dans un système continu de commande de procédé.

26. Procédé selon l'une des revendications 21 à 25, caractérisé en ce que pour réaliser plus rapidement le procédé, après introduction de l'échantillon, le réacteur jetable est soumis à l'action de forces d'évacuation ou de mise sous pressions faibles.

27. Procédé selon l'une des revendications 21 à 26, caractérisé en ce que plusieurs réacteurs jetables sont reliés en série ou en parallèle.

28. Procédé selon la revendication 27, caractérisé en ce qu'il permet la détermination d'allergène.
